# EUROPÄISCHE PATENTSCHRIFT

(11) **EP 2 819 973 B1**
(45) Veröffentlichungstag und Bekanntmachung des Hinweises auf die Patenterteilung: **27.11.2019**
(21) Anmeldenummer: 13709750.7
(22) Anmeldetag: 01.03.2013
(51) Int. Cl.: C04B 35/486, C04B 35/488, C04B 35/626, C04B 35/645, A61L 27/10, A61C 13/00, A61C 5/70

(54) **VERFAHREN ZUR HERSTELLUNG EINES KERAMISCHEN SINTERFORMKÖRPERS AUS Y2O3-STABILISIERTEM ZIRKONOXID**
PROCESS FOR PRODUCING A SHAPED SINTERED CERAMIC BODY COMPOSED OF Y2O3-STABILIZED ZIRCONIUM OXIDE
PROCÉDÉ DE FABRICATION D'UN CORPS MOULÉ FRITTÉ CÉRAMIQUE COMPOSÉ D'OXYDE DE ZIRCON STABILISÉ PAR Y2O3

(30) Priorität: 01.03.2012 DE 102012101741
(43) Veröffentlichungstag der Anmeldung: 07.01.2015
(73) Patentinhaber: Fraunhofer Gesellschaft zur Förderung der angewandten Forschung e.V., 80686 München (DE)
(72) Erfinder: JOHANNES, Martina, 07629 Hermsdorf (DE); SCHNEIDER, Jens, 99425 Weimar (DE)
(74) Vertreter: Oehmke, Volker
(86) Internationale Anmeldenummer: PCT/DE2013/100079
(87) Internationale Veröffentlichungsnummer: WO 2013/127398

(56) Entgegenhaltungen:
- WO-A2-2010/061196
- US-A- 4 506 024
- US-A- 5 958 311
- US-A- 6 087 285
- MUNOZ-SALDANA J. ET AL.: JOURNAL OF MATERIALS RESEARCH, Bd. 18, 2003, Seiten 2415-2426, XP8161148, in der Anmeldung erwähnt
- GUSTAVO SUÁREZ ET AL: "Effect of starting powders on the sintering of nanostructured ZrO 2 ceramics by colloidal processing", SCIENCE AND TECHNOLOGY OF ADVANCED MATERIALS, Bd. 10, Nr. 2, 1. April 2009 (2009-04-01), Seite 025004, XP055058585, ISSN: 1468-6996, DOI: 10.1088/1468-6996/10/2/025004
- SUAREZ ET AL.: JOURNAL OF NANOSCIENCE AND NANOTECHNOLOGY, Bd. 10, 2010, Seiten 6634-6640, XP8161149, in der Anmeldung erwähnt

## Beschreibung

Die Erfindung betrifft ein Verfahren zur Herstellung eines keramischen Sinterformkörpers aus Y₂O₃-stabilisiertem Zirkonoxid, wie diese gattungsgemäß aus der Veröffentlichung von Muñoz-Saldaña J. et al. (2003, Journal of Materials Research 18, 2415-2426) bekannt sind.

Zirkonoxid (ZrO₂) ist eine Keramik, die beispielsweise als Feuerfestkeramik, als technische Keramik und in der Prothetik Verwendung findet. Die Kristallstruktur des Zirkonoxids wird anhand der für das Gebiet der Kristallographie üblicherweise benutzten Kristallsysteme klassifiziert. Diese Kristallsysteme sind beispielsweise bei Hahn, T. (1983: International Table for Christallography, Reidel, Dordrecht) aufgeführt.

Tetragonal stabilisiertes Zirkonoxid (nachfolgend mit TZP abgekürzt) gehört zu den festesten Hochleistungskeramiken auf dem Markt. TZP-Keramiken werden beispielsweise im Maschinenbau, als Biokeramik und für Haushaltsgegenstände verwendet.

Um die tetragonale Phase zu stabilisieren, werden bestimmte Metalloxide wie Y₂O₃ (Yttriumoxid), CaO (Kalziumoxid), MgO (Magnesiumoxid) oder CeO₂ (Ceroxid) in geringen Konzentrationen zudotiert. Die höchsten Festigkeiten werden mit 3 mol-% Y₂O₃ dotiertem Zirkonoxid erreicht. Dieses wird daher auch mit 3Y-TZP bezeichnet. Die tetragonale Phase ist in diesem Material metastabil, d.h. sie ist nicht thermodynamisch stabil, wobei eine Umwandlung in die monokline Phase aber kinetisch gehemmt ist. Bei Raumtemperatur findet eine solche Phasenumwandlung ohne äußeren Einfluss in endlichen Zeiten nicht statt.

Die Festigkeit des 3Y-TZP und anderer Y-TZP ist auf die sogenannte Umwandlungsverstärkung zurückzuführen. Dabei wandelt sich unter Einwirkung mechanischer Spannungen, bevor ein Bruch des Materials eintritt, die tetragonale Phase in die monokline Phase um. Dabei wird Bruchenergie verbraucht und Druckspannungen in dem Material induziert. Diese können einer weiteren Rissausbreitung entgegen wirken. Somit hängt die Festigkeit des Materials von der thermodynamischen Instabilität der tetragonalen Phase bzw. von den die Phasenumwandlung verursachenden Parametern ab.

Die Instabilität der tetragonalen Phase wirkt sich jedoch nicht nur positiv auf das Materialverhalten auf. In feuchter Umgebung, z. B. in Wasser oder in mit Wasserdampf gesättigter Luft, findet die Phasenumwandlung bereits bei Temperaturen < 100°C spontan statt. Dabei wandelt sich die tetragonale Phase spontan in die monokline Phase um. Dieser Prozess wird als hydrothermale Alterung oder als "aging" oder "low temperature degradation" bezeichnet. Die hydrothermale Alterung ist dabei abhängig von der Temperatur und von der Konzentration der Wassermoleküle in der umgebenden Atmosphäre.

Die hydrothermale Alterung beginnt immer an der Oberfläche des Materials und schreitet mit relativ konstanter Geschwindigkeit in das Volumen des Materials fort. Zuerst werden oberflächennahe Bereiche angegriffen, wodurch die Rauigkeit ansteigt und die Härte des Materials abfällt. Besonders bei Bauteilen mit polierten Oberflächen oder bei Verschleißpaarungen werden enorme wirtschaftliche Verluste durch hydrothermale Alterung verursacht. Die Standzeit solcher Bauteile wird deutlich reduziert. Während sich die hydrothermale Alterung bei Bauteilen mit hohem Verhältnis von Volumen zur Oberfläche nicht merklich auf die Bauteilfestigkeit auswirkt, kommt es besonders bei Bauteilen, deren Oberfläche eine spezielle Funktion erfüllt, zu katastrophalen Ausfällen.

Bei dentalen Restaurationen aus 3Y-TZP spielt aus ästhetischen Gründen die Transluzenz des Materials eine wichtige Rolle. Darum ist angestrebt, z. B. Kronen- und Brückengerüste aus 3Y-TZP sowohl mit hoher Transluzenz als auch mit hoher hydrothermaler Beständigkeit herzustellen. Diese Ansprüche ließen sich in der Vergangenheit nur bedingt miteinander vereinen.

In den zurückliegenden zehn Jahren wurden erhebliche Anstrengungen unternommen, die hydrothermale Stabilität von 3Y-TZP zu erhöhen. Die kann prinzipiell durch eine Erhöhung des Stabilisatorgehaltes des Materials; eine homogene Verteilung des Stabilisators in der Matrix des Materials; durch eine Reduzierung der Korngröße sowie durch die Zugabe von Al₂O₃ ≥ 0,25 Ma.-% erreicht werden. Dabei führt die Erhöhung des Gehaltes des Stabilisators zu einer Verschlechterung der mechanischen Eigenschaften. Die Zugabe von Al₂O₃ führt nach Herstellerangaben zu einer erhöhten hydrothermalen Stabilität, jedoch wird dadurch die Transluzenz der Keramik aufgrund von Streuungsphänomen erheblich reduziert.

Es ist bekannt, dass die tetragonale Phase stabilisiert werden kann, wenn die Korngröße unterhalb eines kritischen Wertes liegt. Die kritische Korngröße wird von Muñoz-Saldaña et al. (2003) mit 360 nm, von Chen et al. (1989: Journal of Materials Science 24(2): 453-456) mit 370 nm und von Tsukuma et al. (1984: Science and technology of zirconia 11: 382-390) mit 400 nm angegeben. Andere Autoren berichten, dass die von ihnen präparierte TZP Keramik mit einer Korngröße von 200 nm nicht alterungsstabil war (Kern et al., 2011: Journal of Ceramic Science and Technology 2(3): 147-154).

Durch die Veröffentlichung von Suärez et al. (2009: Science and Technology of Advanced Materials 10(2): 25004) sind Sinterformkörper mit mittleren Korngrößen von etwa 100 nm bekannt, die ausschließlich in der tetragonalen Phase vorliegen und aus Nanopulvern mit Partikelgrößen von 65 nm hergestellt wurden.

Nanomaterialien sind laut der Definition der EU-Kommission vom 18.10.2011 Materialien mit Partikelgrößen unter 100 nm (z. B. Nanopartikel, Nanoplättchen oder Nanofasern).

Aus der Schrift WO 2010/061196 A2 ist die Präparation eines alterungsstabilen 3Y-TZP aus kommerziellen Nanopulvern mit einer Partikelgröße des Nanopulvers von 65 nm bekannt.

Nachteilig an den bekannten Verfahren ist, dass die teuren Nanopulver verwendet werden, so dass eine kostengünstige Produktion alterungsstabiler Sinterformkörper nicht möglich ist.

Aus den Publikationen von Suarez et al. (Suarez et al., 2010, Journal of Nanoscience and Nanotechnology 10: 6634-6640 und Suarez et al., 2009, Science and Technology of advanced Materials 10, doi : 10.1088/1468-6996/10/2/025004) ist die Verwendung von Mahlkörpern von 50 µm Größe bekannt, um Agglomerationen von Nanopulvern wieder aufzulösen. Solche Agglomerationen entstehen, wenn Nanopulver über längere Zeiträume gelagert wird. Die Tendenz von Nanopulvern zur Bildung solcher Agglomerate ist ein weiterer Nachteil bei deren Verwendung.

Die Transmission von sichtbarem Licht ist bei Y-TZP durch die Doppelbrechung des tetragonalen Zirkonoxid stark reduziert. Nach Klimke et al. (2011: Journal of the American Ceramic Society 94 (6): 1850-1858) kann durch die Verringerung der Korngröße auf 150 nm eine theoretische inline-Transmission von ca. 7 % bei einer Wellenlänge von 550 nm gemessen werden. Bei dieser Wellenlänge besitzt das menschliche Auge die höchste Empfindlichkeit.

Muñoz-Saldaña et al. zeigten, dass mit kommerziellen Sub-Mikrometer-Pulver eine alterungsstabile 3Y-TZP-Keramik mit einer mittleren Korngröße von 320 nm und einer 3-Punkt-Biegefestigkeit von 1400 MPa realisierbar sind.

Der Erfindung liegt die Aufgabe zugrunde, eine gegenüber dem Stand der Technik kostengünstigere und verfahrenseffiziente Möglichkeit zur Herstellung von alterungsstabilen Sinterformkörpern vorzuschlagen.

Die Aufgabe wird durch ein Verfahren gemäss Anspruch 1 gelöst.

Das Sub-Mikrometer-Pulver kann weitere Stoffe enthalten. So können in weiteren Ausführungen des erfindungsgemäßen Verfahrens beispielsweise 0,2 - 20 Ma.-% α-Al₂O₃ oder unstabilisiertes Zirkonoxid oder ein Gemisch dieser Stoffe enthalten sein.

Das Zerkleinern des dispergierten Sub-Mikrometer-Pulvers wird vorzugsweise in einer Rührwerkskugelmühle mittels Mahlkörpern mit einem Durchmesser kleiner oder gleich 100 µm durchgeführt. Der Herstellungsprozess beinhaltet die Dispergierung des Pulvers in Wasser unter Zuhilfenahme eines geeigneten Stabilisators bis auf 65-70 Ma.-% Feststoffanteil. Das Sub-Mikrometer-Pulver wird auf eine Partikelgröße d₉₅ zwischen 0,25 und 0,42 µm (gemessen mittels Sedimentationsanalyse) zerkleinert. Dadurch wird eine effiziente Verfahrensführung gewährleistet.

Durch das Zerkleinern werden auch Agglomerate und Aggregate im Sub-Mikrometer-Pulver weitestgehend beseitigt. Durch die in der wässrigen Phase dispergierten Partikel ist ein stabiler Schlicker gebildet, der über mehrere Tage verarbeitbar ist. Moderne instrumentierte Rührwerkskugelmühlen bieten die Möglichkeit, den Energieeintrag während des Mahlprozesses zu verfolgen und somit in Abhängigkeit von der Feststoffmasse reproduzierbare Mahlergebnisse zu erhalten. Moderne Abtrennsysteme erlauben den Einsatz von Kleinstmahlkörpern unter 100 µm Durchmesser. Mit diesen ist es möglich, bei schwach agglomerierten Rohstoffen kleine Partikelgrößen und eine sehr enge Partikelgrößenverteilung im Schlicker zu erreichen. Der Durchsatz der Mühle kann von 80 kg bis zu 150 kg Feststoff pro Stunde betragen und liegt damit in einer industriell nutzbaren Größenordnung.

Das zu dispergierende Sub-Mikrometer-Pulver weist vorzugsweise eine spezifische Oberfläche kleiner als 20 m² / g auf.

Die Formgebung der Dispersion zu einem Körper erfolgt mittels Schlickerguss. Die Formgebung wird mittels Schlickerguss in Gipsformen realisiert. Die Gründichte des gegossenen Formkörpers beträgt > 60 % der theoretischen Sinterdichte, ohne dass der Grünkörper isostatisch gepresst wird.

Das Sintern des Körpers zu dem Sinterformkörper erfolgt bei einer Sintertemperatur zwischen 1200°C und 1350°C, vorzugsweise bei einer Sintertemperatur zwischen 1250°C und 1300°C. Die Sinteraktivität des Pulvers ist nach dem Zerkleinern so hoch, dass der Grünkörper bei den vergleichsweise niedrigen Sintertemperaturen gesintert werden kann und danach eine Sinterdichte von mindestens 95 % der theoretischen Sinterdichte erreicht.

Der Sinterformkörper wird in einer weiteren Ausführung des erfindungsgemäßen Verfahrens bei einer Temperatur zwischen 1200°C und 1350°C, bevorzugt zwischen 1250°C und 1330°C, heiß isostatisch nachverdichtet. Es wird eine Sinterdichte von mindestens 99,0 % der theoretischen Sinterdichte erreicht. Die Berechnung der theoretischen Dichte erfolgt unter Annahme eine Dichte von 6,1 g/cm³ für 3Y-TZP und 3,98 g/cm³ für α-Al₂O₃.

Der so hergestellte Sinterformkörper weist eine Korngröße von <180 nm, vorzugsweise zwischen 150 nm und 170 nm, auf und zeigt keine hydrothermale Alterung nach einer Exposition für 120 Stunden bei 134°C bei 2 bar in Wasserdampfatmosphäre. Die Gefügeeigenschaften des mit dem erfindungsgemäßen Verfahren hergestellten Sinterformkörpers wirken sich dahingehend aus, dass der so hergestellte Sinterformkörper die hohe Alterungsstabilität gegenüber hydrothermalen Angriffen besitzt. Der Anteil monokliner Phase liegt nach 120 Stunden Exposition bei weniger als 3 Ma.-%, vorzugsweise aber bei weniger als 2 Ma.-%.

Die dichten Sinterformkörper aus 100 % 3Y-TZP haben eine 4-Punkt-Biegefestigkeit von > 1000 MPa und eine Mikrohärte HV 0,1 von > 13 GPa. Die Sinterformkörper aus 90 % 3Y-TZP und 10 % α-Al₂O₃ erreichen Festigkeiten von ca. 1700 MPa und eine Mikrohärte HV 0,1 von > 18 GPa.

Die Vorteile des erfindungsgemäßen Verfahrens liegen in der Verwendung von kommerziellem Sub-Mikrometer-Pulver verbunden mit einem optimierten Zerkleinerungsprozess mittels Kleinstmahlkörpern. Dadurch werden Aggregate und Agglomerate optimal zerkleinert und die Partikel in einem hoch konzentrierten Schlicker dispergiert, der für die Formgebung mittels Schlickerguss geeignet ist. Die feinen Partikel liegen in einer sehr engen Verteilung vor, was die Grundlage für eine hohe Sinteraktivität und ein feines und sehr homogenes Gefüge darstellt.

Das erfindungsgemäße Verfahren erlaubt ein hohes Maß an wirtschaftlicher Effizienz bei der Herstellung keramischer Sinterformkörper aus einem Y₂O₃-stabilisierten Zirkonoxid. Dazu tragen die günstigen Rohstoffpreise für Sub-Mikrometer-Pulver bei. Das Aufbereitungsverfahren mittels Rührwerkskugelmühle ist scale-up-fähig und kann ausgehend vom Labormaßstab auf industrielle Standards übertragen werden.

Die Materialeigenschaften des mit dem erfindungsgemäßen Verfahren hergestellten Sinterformkörpers können beispielsweise anhand eines Materialmusters nachvollzogen werden. Dabei können die chemische und kristalline Zusammensetzung, die Korngröße, die Sinterdichte und die Festigkeit des Materials untersucht werden. Durch Mikrostrukturanalyse mittels Rasterelektronenmikroskopie werden die Korngröße und die Art der kontinuierlichen bzw. dispergierten Phasen nachgewiesen. Die chemische Analyse gibt Aufschluss darüber, welche Elemente im Material des Sinterformkörpers vorhanden sind. Anhand der Röntgenphasenanalyse und anschließender Rietveld-Verfeinerung lassen sich die kristalline Zusammensetzung sowie die hydrothermale Beständigkeit der Sinterformkörper quantitativ prüfen. Die Stabilisierung der tetragonalen Phase zur Vermeidung der hydrothermalen Alterung hat auch zur Folge, dass die Umwandlungsverstärkung gehemmt ist. Daraus resultieren für feinstkörnige, aus Nanopulvern hergestellte, Y-TZP-Keramiken Festigkeiten, die, verglichen mit Keramiken aus Sub-Mikrometer-Körnungen, vermindert sind.

Der mit dem erfindungsgemäßen Verfahren hergestellte Sinterformkörper findet dort Anwendung, wo konventionelles Y-TZP aufgrund seiner hydrothermalen Alterungstendenz nur sehr bedingt eingesetzt werden kann. Das betrifft insbesondere medizinische Anwendungen wie Implantate für künstliche Gelenke und Dentalimplantate. Weiterhin besteht eine Verwendungsmöglichkeit für Ventile und Düsen in Verbrennungsmotoren sowie als Katalysatorträger. Auskleidungen von Pumpen für chemisch aggressive Stoffe sowie für die Medienförderung bei erhöhten Temperaturen sind mit diesem Material ebenfalls möglich. Insbesondere ist dieses Material aufgrund seiner guten Oberflächen-Bearbeitbarkeit und hydrothermalen Beständigkeit für Gleitringdichtungen in Mischbatterien geeignet. Der mittels des erfindungsgemäßen Verfahrens hergestellte Sinterformkörper und der erfindungsgemäße Sinterformkörper können jeweils als Rohkörper für Implantate verwendet werden. Dabei ist es grundsätzlich auch möglich, den Sinterformkörper ohne weitere Bearbeitung als Implantat zu verwenden. Er kann als Rohkörper für dentale Kronen- und Brückengerüste verwendet werden. Im Bereich des Anlagen- und Maschinenbaus kann der Sinterformkörper als Bauteil in Pumpen, beispielsweise für Pumpenauskleidungen oder als Bauteil in Turbinen, wobei das Bauteil mindestens bereichsweise mit Wasserdampf in Berührung kommen kann, verwendet werden. Auch ist es möglich, den Sinterformkörper als Bauteil in Verbrennungsmotoren zu verwenden, wobei das Bauteil mindestens über Bereiche seiner Oberfläche mit flüssigen Kraftstoffen, Lösungsmitteln, Lösungsmittelgemischen, Wasser, Wasserdampf oder deren Gemischen, Dämpfen und Aerosolen in Berührung kommen kann. Vorteilhaft sind weitere Verwendungen, bei denen der Sinterformkörper unter Bedingungen eingesetzt wird, die eine hydrothermale Alterung begünstigen.

In einer weiteren Ausführung des Sinterformkörpers, der mit dem erfindungsgemäßen Verfahren hergestellt wurde, enthält dieser 2 - 15 Ma.-% unstabilisiertes Zirkonoxid. Das unstabilisierte Zirkonoxid liegt vorzugsweise in der tetragonalen Phase vor.

Durch die teilweise Substitution von 3 mol-% Y₂O₃-stabilisiertem Zirkonoxid durch unstabilisiertes Zirkonoxid sinkt der effektive Stabilisatorgehalt in der Zirkonoxid-Phase, was zu einer Steigerung der Festigkeit und der Bruchzähigkeit führen kann.

Der Zirkonoxid-Anteil des mit dem erfindungsgemäßen Verfahren hergestellten Sinterformkörpers liegt zu 75 - 95 Ma.-% in der tetragonalen Phase und zu 5 - 25 Ma.-% in der kubischen Phase vor. Ein monokliner Zirkonoxid-Anteil < 2 Ma.-% kann vorhanden sein. Der Zirkonoxid-Anteil umfasst Y₂O₃-stabilisiertes Zirkonoxid und unstabilisiertes Zirkonoxid. Die Phasenzusammensetzung kann aus Röntgenbeugungsdaten mit Hilfe der Rietveld-Verfeinerung bestimmt werden.

Eine weitere und für eine spezielle Anwendung des mit dem erfindungsgemäßen Verfahren hergestellten Sinterformkörpers, beispielsweise für dentale Implantate und Aufbauten, vorteilhafte Ausführung des Sinterformkörpers ist dann gegeben, wenn eine optische Transmission bei einer Wellenlänge von 550 nm und einer Dicke von 0,5 mm des Materials des Sinterformkörpers mindestens 7 % beträgt. Die optische Transmission wird hier vereinfacht für eine Wellenlänge von 550 nm angegeben, bei der das Auge die größte Empfindlichkeit bei Tageslicht besitzt.

Die Erfindung ist nachfolgend anhand von Ausführungsbeispielen, Abbildungen und Tabellen näher erläutert. Es zeigen:
Fig. 1 eine erste Tabelle, deren Inhalt Zusammensetzungen und Mahlparameter von untersuchten Sinterkörpern ist,
Fig. 2 Partikelgrößen d50 und d95, die nach einer Mahlung eines Sub-Mikrometer-Pulvers gemäß dem erfindungsgemäßen Verfahren im Schlicker gemessen wurden,
Fig. 3 Sinterkurven gegossener Probekörper mit Angaben der HIP-Temperatur (Pfeile) und der relativen Dichte nach der HIP (Heißisostatisches Pressen) (grau gefüllte Symbole),
Fig. 4 FESEM-Aufnahmen von Teilen von herkömmlichen (erste Teilabbildung, Probe Z-1) und mit dem erfindungsgemäßen Verfahren hergestellten Sinterkörpern (zweite und dritte Teilabbildung, Proben Z-2 bzw. ZA-10),
Fig. 5 Korngrößen der Sinterkörper,
Fig. 6 Alterungsstabilität der Sinterkörper ermittelt durch beschleunigte Alterungsversuche im Autoklaven bei 134°C und 2 bar in Wasserdampf,
Fig. 7 eine zweite Tabelle, deren Inhalt Struktureigenschaften untersuchter Sinterkörper ist,
Fig. 8 eine dritte Tabelle, deren Inhalt mechanische Eigenschaften untersuchter Sinterkörper nach einer Bearbeitung mittels HIP ist,
Fig. 9 4-Punkt Biegefestigkeiten und Weibull-Module von untersuchten Sinterkörpern.

Beispiel 1: In einem Rühraggregat werden 270 ml Wasser vorgelegt und ein geeigneter Dispergator zugegeben. Anschließend werden 500 g 3Y-TZP Pulver mit einer spezifischen Oberfläche von 6 m²/g eingerührt. Der Schlicker wird auf eine Rührwerkskugelmühle aufgegeben. Die Mühle ist zu 85 % mit Mahlkugeln mit einem Durchmesser von 100 µm gefüllt. Der Schlicker wird zwei Stunden bei 3500 U/min, das entspricht einer Umfangsgeschwindigkeit von 11 m/s, gemahlen bis eine Partikelgrößenverteilung vorliegt die dadurch gekennzeichnet ist, dass der d₉₅-Wert < 0,42 µm und der d₅₀-Wert < 0,3 µm beträgt.

Die Mahldauer beträgt für diese Feststoffmenge ca. zwei Stunden. Der Schlicker wird in Gipsformen gegossen, die in ihren Abmessungen bereits ein Schwindungsaufmaß eingerechnet haben. Die Standzeit wird durch die Größe des Bauteils bestimmt. Die Grünkörper werden bei 50°C an Luft getrocknet und anschließend zwei Stunden bei 1250°C gesintert. Die gesinterten Formkörper weisen ca. 95 % der theoretischen Dichte auf. Die Formkörper werden anschließend bei 1250°C heiß isostatisch gepresst und weisen schließlich eine Dichte von 6,07 g/cm³ auf. Das entspricht 99,5 % der theoretischen Dichte.

Die mittlere Korngröße wird mittels Linienschnittverfahren nach DIN EN 623-3 bestimmt und beträgt für dieses Material 160 nm. Die Festigkeit beträgt 1063 MPa und die Mikrohärte 18 GPa. Die Sinterformkörper werden 120 Stunden bei 134°C in Wasserdampfatmosphäre gealtert. Der monokline Anteil nach der Alterung wird mittels XRD (X-Ray Diffraction) bestimmt. Die Proben weisen ein Gehalt an monokliner Phase zwischen 0,5 % und 3 % auf.

Beispiel 2: In einem Rühraggregat werden 270 ml Wasser vorgelegt und ein geeigneter Dispergator zugegeben. Anschließend werden 450 g 3Y-TZP-Pulver und 50 g α-Al₂O₃-Pulver mit einer spezifischen Oberfläche von 6 m²/g bzw. 12 m²/g eingerührt. Der Schlicker wird auf eine Rührwerkskugelmühle aufgegeben.

Die Mühle ist zu 85 % mit Mahlkugeln mit einem Durchmesser von 100 µm gefüllt. Der Schlicker wird zwei Stunden bei 3500 U/min, das entspricht einer Umfangsgeschwindigkeit von 11 m/s, gemahlen bis eine Partikelgrößenverteilung vorliegt die dadurch gekennzeichnet ist, dass der d₉₅-Wert < 0,42 µm und der d₅₀-Wert < 0,30 µm beträgt. Die Mahldauer beträgt für diese Feststoffmenge ca. zwei Stunden. Der Schlicker wird in Gipsformen gegossen, die in ihren Abmessungen bereits ein Schwindungsaufmaß eingerechnet haben. Die Standzeit wird durch die Größe des Bauteils bestimmt. Die Grünkörper werden bei 50°C an Luft getrocknet und anschließend zwei Stunden bei 1300°C gesintert. Die gesinterten Formkörper weisen ca. 95 % der theoretischen Dichte auf. Die Formkörper werden anschließend bei 1300°C heiß isostatisch gepresst und weisen schließlich eine Dichte von 5,76 g/cm³ auf. Das entspricht 99,5 % der theoretischen Dichte von 5,79 g/cm³.

Die mittlere Korngröße wird mittels Linienschnittverfahren nach DIN EN 623-3 bestimmt und beträgt für dieses Material 143 nm. Die Festigkeit beträgt 1700 MPa. Die Sinterformkörper werden 120 Stunden bei 134°C in Wasserdampfatmosphäre gealtert. Der monokline Anteil nach der Alterung wird mittels XRD bestimmt. Die Proben weisen einen Gehalt an monokliner Phase zwischen 0 und 1 % auf.

Beispiel 3: Es wurden zwei 3Y-TZP Chargen und eine ATZ Charge (90 wt.-% 3Y-TZP / 10 wt.-% Al₂O₃) präpariert. Als Rohstoffe wurden TZ3Y-SE (Tosoh, Japan) und TM-DAR (Taimei Chemicals, Japan) mit Partikelgrößen von 70 nm bzw. 100 nm verwendet. Die Pulver wurden unter Verwendung von 0,5% Poly-Ammoniumacrylat (Zschimmer und Schwarz, Germany) in Wasser dispergiert und anschließend in einer Rührwerkskugelmüle (Mini Cer, Netzsch FMT, Germany) unter Verwendung unterschiedlicher Mahlkugeldurchmesser gemahlen und dispergiert. In Tabelle 1 sind die Zusammensetzungen und die Mahlparameter aufgeführt.

Die Partikelgrößenverteilung im Schlicker wurde mit einem Utrafine Particle Analyser (UPA, Microtrac, USA) analysiert. Die Formgebung zu Scheiben mit der Abmessung von 3x20x30 mm erfolgte mittels Schlickerguss in Gipsformen. Anschließend wurden Sinterkurven erstellt und daraus die HIP-Temperaturen abgeleitet. Die Dichte der gehipten Probekörper wurde mit der Archimedes-Methode bestimmt. Die Oberflächen der Scheiben wurden mit Diamantpaste poliert. Die Rauigkeiten Rₐ der Oberflächen lagen zwischen 8,5 und 16 nm. Die Gefüge wurden mit einem FESEM (Zeiss Ultra 55+; Carl Zeiss NTS Germany) untersucht. Die mittlere Korngröße wurde mit dem Linienschnittverfahren ermittelt.

Die Proben wurden in einem Autoklav bei 134°C und 2 bar in Wasserdampf bis zu 200 h gealtert. Die Phasenzusammensetzung der gealterten Proben wurde mittels XRD (D8 Advance, Bruker, Germany) gemessen und mit der Rietveldverfeinerung (AutoQuan, GE-Sensing Technology, Ahrensburg, Germany) quantifiziert.
Die 4-Punkt-Biegefestigkeit wurde an Biegestäben der Größe 2x2,5x25 mm nach EN 843-1 durchgeführt. Es wurden 15 Biegestäbe getestet, anschließend die mittlere Biegefestigkeit σ₀ und der Weibull-Modul m ermittelt.

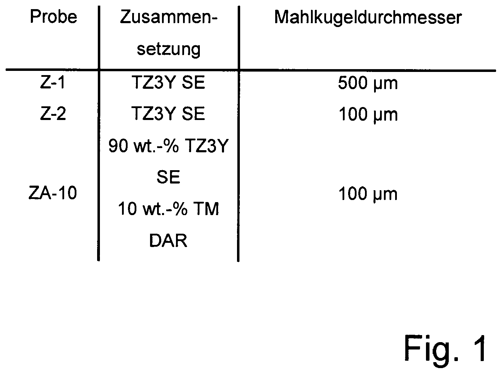

Die Proben Z-1 wurde mit einer Standardprozedur und die Proben Z-2 und ZA-10 in einem optimierten Prozess aufbereitet (Tabelle 1). Obwohl die Mahlenergie bei den Chargen Z-2 und ZA-10 um den Faktor 8 größer ist als bei der Charge Z-1 konnten kleinere Partikelgrößen im Schlicker gemessen werden, was in Abb. 1 dargestellt ist.

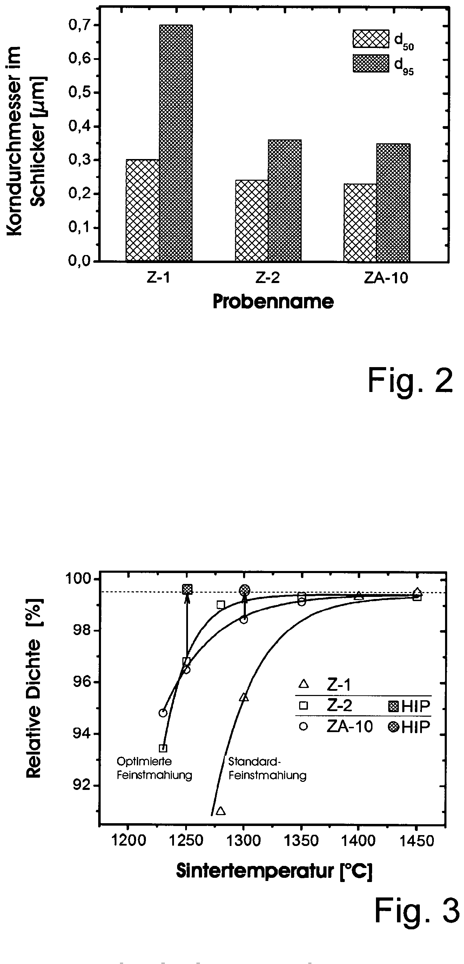

Durch die optimierte Aufbereitung konnte der d₉₅ Wert und damit die Partikelgrößenverteilung im Schlicker deutlich reduziert werden. Daraus resultiert eine erhebliche Steigerung der Sinteraktivität, was anhand der Sinterkurven in Abb. 2 deutlich wird.

Die Probekörper Z-1 wurden bei 1450°C ohne anschließendes HIP gesintert. Die Probekörper Z-2 und ZA-10 wurden bei 1250°C bzw. 1300°C gesintert und bei derselben Temperatur gehipt. Die relativen Dichten aller Proben lagen über 99,5 % der theoretischen Dichten von 6,1 g/cm³ für 3Y-TZP bzw. 5,79 g/cm³ für ATZ 90/10. Die Mikrostruktur der gesinterten bzw. gehipten Probekörper ist in Abb. 2 dargestellt.

In Abb. 2 ist der Effekt der optimierten Aufbereitung deutlich zu erkennen. Die Korngrößen können durch eine optimierte Aufbereitung und die dadurch verbundene Erhöhung der Sinteraktivität deutlich verringert werden. Die Korngrößen der Probekörper sind in Abb. 3 dargestellt.

Die Korngrößen in den dichtgesinterten Keramiken verhalten sich proportional zum d₉₅ Wert der Partikelgrößenverteilung im Schlicker (vergl. Abb. 1). Die Partikelgrößenverteilung im Schlicker ist demnach eine Schlüsselgröße für die Optimierung von feinstkörnigen Gefügen.

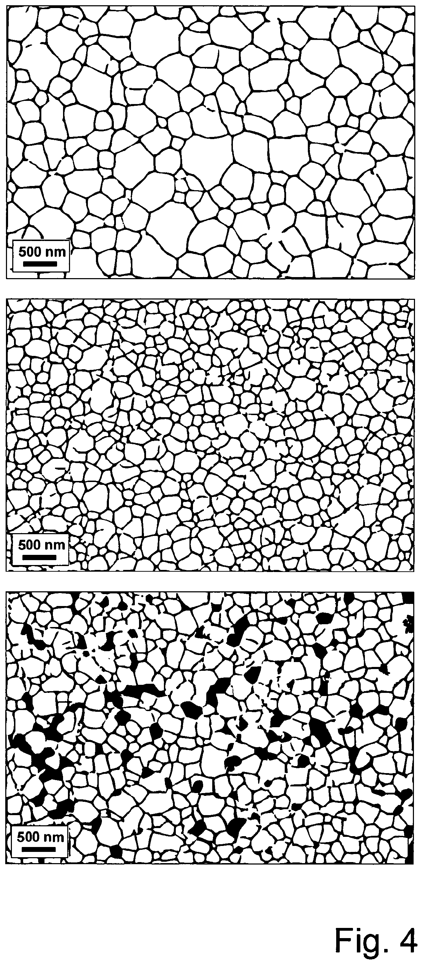

Die Alterungsstabilität der Keramiken wurde in beschleunigten Alterungsversuchen im Autoklaven getestet. In Abb. 4 ist der Gehalt der monoklinen Phase in den Proben in Abhängigkeit von der Alterungszeit bei 134°C in Wasserdampf ist dargestellt.

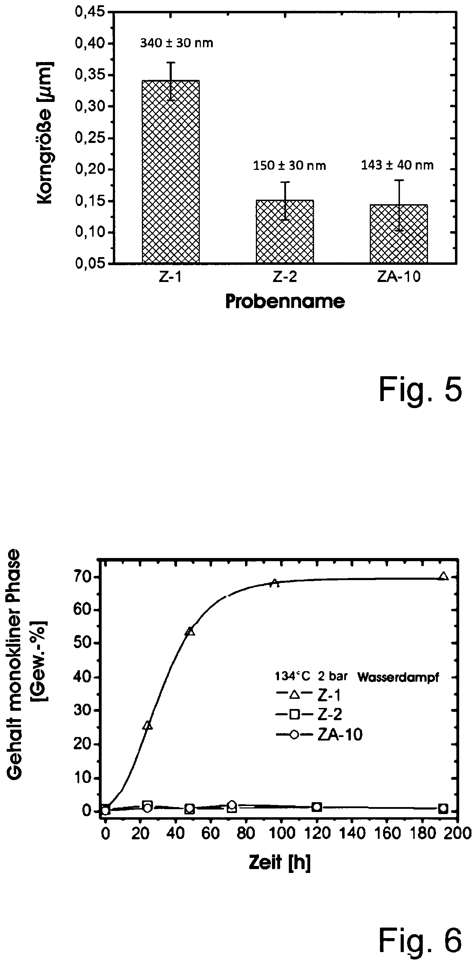

Die Probe Z-1 zeigte einen sehr raschen Anstieg der monoklinen Phase nach kurzen Alterungszeiten. Die Proben Z-2 und ZA-10 zeigen keinen Anstieg der monoklinen Phase im untersuchten Zeitraum. Die Keramiken können demnach als alterungsstabil bezeichnet werden. Als Ursache dafür wird die Stabilisierung der tetragonalen Phase durch die geringe Korngröße vermutet. Dies könnte auch Auswirkungen auf die Festigkeits-Eigenschaften der feinstkörnigen 3Y-TZP-Keramiken haben.
Die 4-Punkt-Biegefestigkeit und der Weibull-Modul der 3Y-TZP und der ATZ-Keramik sind in Abb. 5 dargestellt.

Die Festigkeit der Z-2 Keramik ist gegenüber der Z-1 Keramik reduziert. Es wird vermutet, dass dieser Effekt ebenfalls auf die Stabilisierung der tetragonalen Phase zurückzuführen ist, wodurch die Umwandlungsverstärkung ebenso wie die hydrothermal induzierte Phasentransformation gehemmt ist.

Die ATZ Keramik ZA-10 weist im Gegensatz dazu eine sehr hohe Festigkeit von 1700 MPa und einen Weibull-Modul von 14,3 auf. Die Zugabe von Al₂O₃ zur Y-TZP Matrix kompensiert den negative Einfluss der kleinen Korngröße und führt sogar zu höheren Festigkeiten im Vergleich mit konventionell hergestellter 3Y-TZP Keramik. Die Ursache dafür wird in einer mechanischen Verspannung der Körner aufgrund des thermal mismatch vermutet. Rund um die Al₂O₃ Körner baut sich beim Abkühlen nach dem Sintern eine lokale Ring-Zugspannung in der Y-TZP-Matrix auf, wodurch die treibende Kraft für die Phasenumwandlung lokal erhöht wird und für eine höhere Festigkeit der Keramik sorgen kann.
Daraus resultiert eine hochfeste alterungsstabile Dispersionskeramik, die für den Einsatz als bioinerter Implantat-Werkstoff hervorragend geeignet ist.

In Fig. 1 ist eine Tabelle angegeben, aus der die als Ausgangsstoff für ein erfindungsgemäßes Verfahren verwendetes Sub-Mikrometer-Pulver, dessen Zusammensetzung sowie der Durchmesser der für die Zerkleinerung des jeweiligen Sub-Mikrometer-Pulvers verwendeten Mahlkörper (Mahlkugeln) aufgezeigt sind.

Fig. 2 zeigt die nach der Mahlung im Schlicker enthaltenen und gemessenen Partikelgrößen in den Standardangaben d₅₀ und d₉₅. Diese Größenverteilungen der Partikel wurde durch die Verwendung von Mahlkörpern 500 µm (Probe Z-1) bzw. von 100 µm (Proben Z-2 und ZA-10) erreicht.

In Fig. 3 sind resultierende Sinterkurven gegossener Probekörper der wie oben angegeben gemahlenen Sub-Mikrometer-Pulver gezeigt. Die relative Dichte der erhaltenen Sinterkörper ist in Prozent (Relative Density [%]) angegeben und über der Sintertemperatur in °C (Sintering Temperature [°C]) aufgetragen. Die Temperaturen des Heißisostatischen Pressens (Pfeile) und die damit erreichten relativen Dichten (grau gefüllte Symbole) sind gezeigt.

Fig. 4 zeigt FESEM-Abbildungen der Gefüge der erhaltenen Sinterformkörper der Proben Z-1 (oberste Teilabbildung), Z-2 (mittlere Teilabbildung) und ZA-10 (untere Teilabbildung). Die hellen Körner sind Zirkonium, die dunklen Körner sind Aluminium.

Fig. 5 gibt die Korngrößen der erhaltenen Sinterformkörper an. Die Korngrößen der mittels Mahlkugeln von 100 µm Durchmesser zerkleinerten Sub-Mikrometer-Pulver liegen wesentlich unter der Korngröße des mit Mahlkugeln eines Durchmesser von 500 µm zerkleinerten Sub-Mikrometer-Pulvers. Die Korngröße (Grain Size [µm]) µm ist über den Proben (Sample Name) aufgetragen.

In der Fig. 6 ist der Anteil monokliner Phase in Gewicht-% (Monoclinic Phase Content [wt.-%]) über der Zeit (Aging Time [h]) aufgetragen. Die Sinterformkörper wurden unter einem Druck von 2 bar und Wasserdampf bei 134 °C getestet. Es ist eindeutig zu erkennen, dass die Sinterkörper Z-2 und ZA-10 weit über 120 Stunden hinaus (mindestens bis 192 Stunden) keine Zunahme des Anteils monokliner Phase aufweisen. Das bedeutet, dass die Sinterformkörper Z-2 und ZA-10 über mindestens 192 Stunden unter Wasserdampfatmosphäre alterungsstabil sind. Z-1 zeigt dagegen einen massiven und steilen Anstieg des Anteils monokliner Phase auf über 60 Ma.-% und ist nicht stabil gegenüber hydrothermaler Alterung.

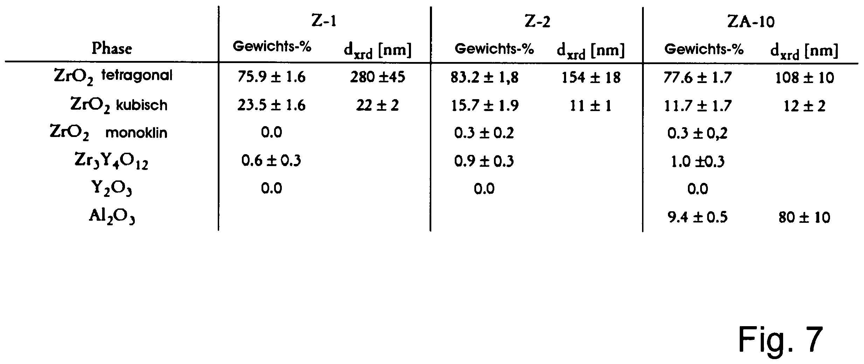

Fig. 7 zeigt eine zweite Tabelle, in der stoffliche Zusammensetzungen der dichten Sinterformkörper aufgeführt sind. Der Fehler ist als dreifache Standardabweichung angegeben (gemäß Rietveld-Programm).

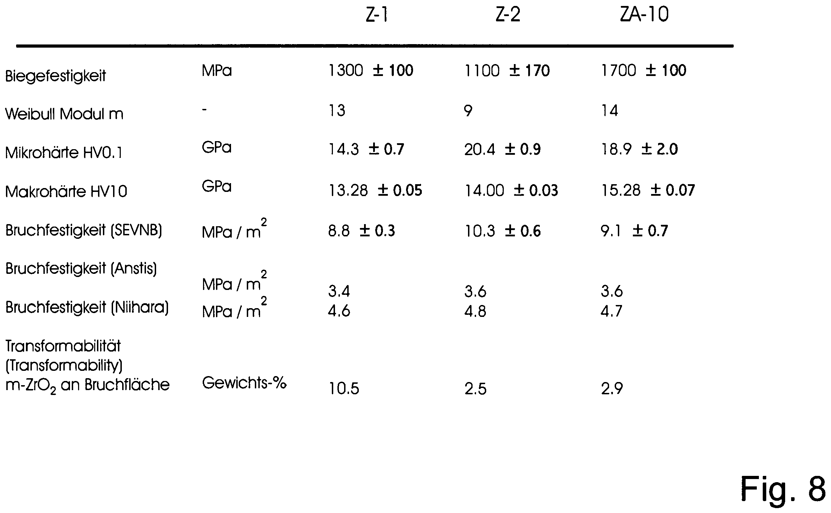

In Fig. 8 ist eine dritte Tabelle gezeigt, in der mechanische Eigenschaften der Sinterformkörper nach einer Behandlung mittels HIP aufgelistet sind. Die Eigenschaften sind: Biegefestigkeit (Bending Strength), Weibull Modul (Weibull Modulus m), Mikrohärte HV0.1 (Microhardness HV0.1), Mikrohärte HV10 (Microhardness HV10), Bruchfestigkeit nach SEVNB (Fracture Toughness (SEVNB)), Bruchfestigkeit nach Anstis (Fracture Toughness (Anstis)), Bruchfestigkeit nach Niihara (Fracture Toughness (Niihara)) und Transformität von m-ZrO₂ auf der Bruchoberfläche (Transformability (m-Zr O₂ on the fracture surface).

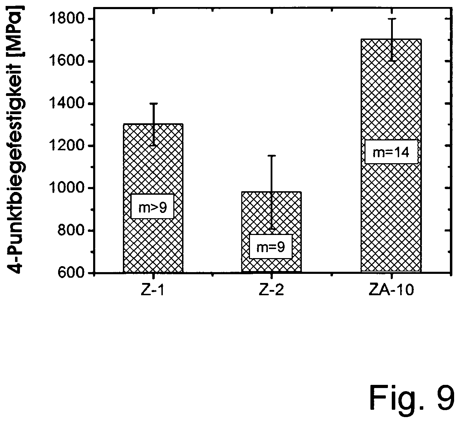

Fig. 9 schließlich zeigt die 4-Punkt-Biegefestigkeit (4-Point Bending Strength [MPa]) der Sinterformkörper Z-1, Z-2 und ZA-10 sowie das jeweils zugehörige Weibull-Modul (Angabe im Kasten auf der jeweiligen Säule).

## Patentansprüche

1. Verfahren zur Herstellung eines keramischen Sinterformkörpers aus 3 Mol.-% Y₂O₃-stabilisiertem Zirkonoxid (3Y-TZP), der 0,2 bis 20 Ma.-% α-Al₂O₃ und/oder 2 bis 15 Ma.-% unstabilisiertes Zirkonoxid enthalten kann, mit den Schritten:
- Dispergieren eines Sub-Mikrometer-Pulvers mit einem Anteil von mindestens 65 Ma.-% des Y₂O₃-stabilisiertem Zirkonoxids in Wasser unter Zuhilfenahme eines geeigneten Stabilisators zu einem Schlicker mit einem Feststoffgehalt von 65 Ma-% bis zu 70 Ma-%,
- Zerkleinern des dispergierten Sub-Mikrometer-Pulvers mittels Mahlkörpern mit einem Durchmesser von kleiner oder gleich 100 µm auf folgende Partikelgrößen:
- d95 von 0,25 µm bis kleiner 0,42 µm, gemessen mittels Sedimentationsanalyse,
- Formgebung der Dispersion des zerkleinerten Sub-Mikrometer-Pulvers mittels Schlickerguss in Gipsformen zu einem Körper, wobei die Gründichte ohne isostatisches Pressen des Körpers größer 60% der theoretischen Sinterdichte ist, und
- Sintern des Körpers zu dem Sinterformkörper.

2. Verfahren nach Anspruch 1, **dadurch gekennzeichnet, dass** das Sub-Mikrometer-Pulver eine spezifische Oberfläche kleiner als 20 m² / g aufweist.

3. Verfahren nach einem der Ansprüche 1 bis 2, **dadurch gekennzeichnet, dass** das Zerkleinern des dispergierten Sub-Mikrometer-Pulvers in einer Rührwerkskugelmühle durchgeführt wird.

4. Verfahren nach einem der Ansprüche 1 bis 3, **dadurch gekennzeichnet, dass** der Körper bei einer Sintertemperatur zwischen 1200°C und 1350°C, insbesondere zwischen 1250°C und 1300°C, zu dem Sinterformkörper gesintert wird.

5. Verfahren nach Anspruch 4, **dadurch gekennzeichnet, dass** der Sinterformkörper bei einer Temperatur zwischen 1200°C und 1350°C durch heißisostatisches Pressen nachverdichtet wird.

## Claims

1. Process for the production of a ceramic sintered shaped body containing 3 mol% Y₂O₃-stabilized zirconia (3Y-TZP), which sintered shaped body might contain 0.2 to 20 mass% of α-Al₂O₃ and/or 2 to 15 mass% of unstabilized zirconia, including the steps of:
- dispersion of a submicron powder with a fraction of at least 65 mass% of the Y₂O₃-stabilized zirconia in water with the aid of a suitable stabilizer to form a slurry with a solids content of 65 mass% to 70 mass%,
- comminution of the dispersed submicron powder by means of grinding media having a diameter of less than or equal to 100 µm to the following particle sizes:
- d95 of 0.25 µm to less than 0.42 µm, measured by means of sedimentation analysis,
- shaping of the dispersion of the comminuted submicron powder by means of slipcasting in plaster molds to form a body, wherein the green density is more than 60% of the theoretical sintered density without isostatic pressing of the body, and
- sintering of the body to form the sintered shaped body.

2. Process according to claim 1, **characterized in that** the submicron powder has a specific surface area of less than 20 m²/g.

3. Process according to one of claims 1 to 2, **characterized in that** the comminution of the dispersed submicron powder is carried out in an agitator ball mill.

4. Process according to one of claims 1 to 3, **characterized in that** the body is sintered at a sintering temperature of between 1200 °C and 1350 °C, particularly between 1250 °C and 1300 °C, to form the sintered shaped body.

5. Process according to claim 4, **characterized in that** the sintered shaped body is repressed by hot isostatic pressing at a temperature of between 1200 °C and 1350 °C.

## Revendications

1. Procédé de production d'un corps fritté céramique contenant 3 % en moles de zircone stabilisée à l'Y₂O₃ (3Y-TZP), le corps fritté pouvant contenir 0,2 à 20 % en masse de α-Al₂O₃ et/ou 2 à 15 % en masse de zircone non stabilisée, comprenant les étapes consistant à:
- disperser une poudre submicrométrique avec une fraction d'au moins 65 % en masse de la zircone stabilisée à l'Y₂O₃ dans l'eau à l'aide d'un stabilisant approprié pour former une barbotine avec une teneur en matières solides de 65 % en masse à 70 % en masse,
- broyer la poudre submicrométrique dispersée au moyen de corps de broyage avec un diamètre inférieur ou égal à 100 µm aux tailles des particules suivantes:
- d95 de 0,25 µm à inférieures à 0,42 µm, mesurées par analyse de sédimentation,
- mouler la dispersion de la poudre submicrométrique broyée par coulée en barbotine dans des moules en plâtre pour former un corps, la densité verte étant supérieure à 60 % de la densité frittée théorétique sans pression isostatique, et
- fritter le corps pour former un corps fritté.

2. Procédé selon la revendication 1, **caractérisé en ce que** la poudre submicrométrique a une surface spécifique inférieure à 20 m²/g.

3. Procédé selon une des revendications 1 à 2, **caractérisé en ce que** le broyage de la poudre submicrométrique dispersée est exécuté dans un broyeur agitateur à billes.

4. Procédé selon une des revendications 1 à 3, **caractérisé en ce que** le corps est fritté à une température de frittage d'entre 1200 °C et 1350 °C, particulièrement entre 1250 °C et 1300 °C, pour former un corps fritté.

5. Procédé selon la revendication 4, **caractérisé en ce que** le corps fritté est densifié par pression isostatique à chaud à une température d'entre 1200 °C et 1350 °C.
